# EUROPEAN PATENT SPECIFICATION

(11) **EP 2 128 616 B1**
(45) Date of publication and mention of the grant of the patent: **28.09.2016**
(21) Application number: 08721270.0
(22) Date of filing: 04.03.2008
(51) Int. Cl.: G01N 33/543, G01N 33/553, C07K 17/00

(54) **SUPPORT HAVING PROTEIN IMMOBILIZED THEREON AND METHOD OF PRODUCING THE SAME**
SUBSTRAT MIT IMMOBILISIERTEM PROTEIN UND VERFAHREN ZU DESSEN HERSTELLUNG
SUPPORT AYANT UNE PROTÉINE IMMOBILISÉE SUR CELUI-CI ET SON PROCÉDÉ DE PRODUCTION

(30) Priority: 28.03.2007 JP 2007084466
(43) Date of publication of application: 02.12.2009
(73) Proprietor: JSR Corporation, Minato-ku, Tokyo 105-8640 (JP)
(72) Inventor: KATAYOSE, Satoshi, Tokyo 105-8640 (JP); FUKUTA, Tetsuo, Tokyo 105-8640 (JP); MURATA, Mitsuhiro, Tokyo 105-8640 (JP)
(74) Representative: TBK
(86) International application number: PCT/JP2008/053850
(87) International publication number: WO 2008/117638

(56) References cited:
- WO-A1-2004/101730
- WO-A1-2005/089933
- JP-A- 2004 170 195

## Description

### TECHNICAL FIELD

The present invention relates to a method of producing a protein-immobilized support that can efficiently immobilize a protein on a support, and a protein-immobilized support on which a protein is immobilized.

### BACKGROUND ART

A support on which a protein is immobilized (i.e., protein-immobilized support) has been used to purify or detect a biological substance or a chemical substance utilizing the protein as a probe. For example, a support on which Protein A or Protein G (i.e., a protein that has affinity for an antibody molecule) is immobilized has been used for antibody affinity purification. A support on which an antibody is immobilized has been used as a diagnostic reagent used to detect and determine the antigen utilizing an antigen-antibody reaction.

When using a protein-immobilized support for such a purification or detection process, it is necessary to increase the amount of protein immobilized on the support in order to increase the purification capability and the detection sensitivity (i.e., performance indices) of the support.

A protein is generally bound to a support by coupling a support having an active functional group (e.g., carboxyl group or tosyl group) to an amino group of the protein molecule. However, a sufficient amount of protein may not be immobilized depending on the molecular species of protein when the content of amino acid having an amino group is low, or the number of amino groups that are present on the surface of the protein conformation that easily comes in contact with the support is small, for example. Moreover, when an amino group that plays an important role in achieving the function and the activity of a protein is consumed due to binding with the support, the function and the activity of the protein immobilized on the support may be lost.

In recent years, a method that evaluates molecular species that interact with a biomaterial derived from a patient or a medicine candidate low-molecular-weight compound using a protein chip or an antibody chip on which various proteins or antibodies are immobilized has been developed as an application of a protein-immobilized support. When producing a protein chip or an antibody chip, a reduction in variation in immobilization amount between proteins is desired in order to facilitate signal analysis.

WO2005089933 (A1) discloses a process for covalently binding a tagged protein to a polymer particle comprising: contacting a tagged protein with a chelating agent-polymer particle conjugate, wherein said tag comprises at least two histidine residues and at least two lysine residues and said chelating agent is tridentate, tetradentate or pentadentate and comprises at least two carboxyl groups and is coordinated by Co²⁺ ions, to form particle-chelating agent Co²⁺ complex: contacting said complex with a carbodiimide; and optionally removing the Co²⁺ ions.

JP2004170195 (A) describes a protein immobilization method including a first process for activating a reactive group in an immobilization carrier with the reactive group having covalent bonding to the immobilized protein with a tag and a second process for reacting a solution containing the immobilized protein to the immobilization carrier after the first process. In the second process, the protein is immobilized to the immobilization carrier through an interaction between the tag and a tag bonding region of the immobilization carrier and the covalent bonding between the reactive group and the protein.

WO2004101730 (A1) mentions a device for analyzing an analyte, which device comprises, inter alia, various means for moving analytes and other items to facilitate binding between analytes and their binding reagents immobilized on a surface and to facilitate clearance of undesirable items away from analyte-binding reagent interaction area to reduce background noise in the assay.

### DISCLOSURE OF THE INVENTION

An object of the invention is to provide a method of producing a protein-immobilized support that can efficiently immobilize a protein that has been difficult to immobilize on a support in a sufficient amount to increase the amount of protein immobilized on a support, and a protein-immobilized support obtained by this production method.

The inventors of the invention conducted extensive studies in order to achieve the above object. As a result, the inventors found that the amount of protein immobilized on a support can be increased, even when using a protein that has a poor immobilization reaction efficiency and cannot be immobilized in a sufficient amount, by utilizing a protein having a tag sequence that includes a basic amino acid molecule. This finding has led to the completion of the invention.

According to one aspect of the invention, there is provided a method of producing a protein-immobilized support as defined in claim 1 comprising immobilizing a protein having a tag sequence on a support, the tag sequence including a sequence that includes three or more consecutive basic amino acids.

In the above method of producing a protein-immobilized support, the basic amino acid are lysine, arginine, or histidine.

In the above method of producing a protein-immobilized support, the tag sequence may be a histidine-tag.

In the above method of producing a protein-immobilized support, the support has at least one functional group selected from a carboxyl group, an epoxy group, and a tosyl group.

In the above method of producing a protein-immobilized support, the support may be magnetic particles.

According to another aspect of the invention, there is provided a protein-immobilized support as defined in claim 6 obtained by the above method.

According to the above method of producing a protein-immobilized support, even when using a protein that has a poor immobilization reaction efficiency and cannot be immobilized in a sufficient amount, the amount of protein immobilized on the support can be increased, for example. Therefore, a large amount of protein is immobilized on the protein-immobilized support obtained by the above method of producing a protein-immobilized support.

### BEST MODE FOR CARRYING OUT THE INVENTION

A protein-immobilized support according to one embodiment of the invention and a method of producing the same are described below.

### 1. Protein-immobilized support and method of producing the same

A method of producing a protein-immobilized support according to one embodiment of the invention is defined in claim 1 and includes immobilizing a protein having a tag sequence (hereinafter referred to as "tagged protein") on a support, the tag sequence including a sequence that includes three or more consecutive basic amino acids.

A protein-immobilized support according to one embodiment of the invention is defined in claim 6 and includes a tagged protein and a support, the tagged protein and the support being chemically bound via a functional group (e.g., imino group or amide bond) that is bound to a tag sequence.

### 1.1. Tagged protein

In the method of producing the protein-immobilized support according to this embodiment, the tagged protein immobilized on the support has a tag sequence including a sequence that includes three or more consecutive basic amino acids.

The number of amino acids included in the tag sequence is three or more (i.e., the upper limit is not particularly limited). The number of amino acids included in the tag sequence is preferably 5 to 30 so that the properties of the protein are not affected.

It is preferable that the tag sequence partially or entirely include basic amino acids. The tag sequence includes three or more consecutive basic amino acids selected from lysine, arginine, and histidine. The tag sequence includes three or more consecutive amino acids that are lysine, arginine, or histidine. It is preferable that the tag sequence includes three to ten consecutive amino acids that are lysine, arginine, or histidine.

When the tag sequence includes only one type of amino acid selected from lysine, arginine, and histidine, the tag sequence is oligolysine, oligoarginine, or oligohistidine. Oligohistidine formed of a pentamer or a hexamer is generally referred to as a histidine-tag (His-tag). It is preferable to use the histidine-tag as the tag sequence since a commercially available expression vector can be used so that a recombinant protein can be easily produced.

The tag sequence may be added to the protein by incorporating the tag sequence in the primary sequence of the protein, or grafting the tag sequence on the amino acid residue of the protein, for example.

When incorporating the tag sequence in the primary sequence of the protein, it is preferable to add the tag sequence to the N-terminal or the C-terminal of the protein molecule in order to minimize the effects on the properties of the protein. Note that the tag sequence may be incorporated in the internal sequence of the protein. In this case, the tagged protein may be prepared by preparing an expression vector provided with a sequence in which a gene that encodes the protein and a gene that encodes the tag sequence are fused in a state in which the open reading frames coincide, culturing *Escherichia coli* or the like that is transformed by the expression vector, and separating and purifying the expressed tagged protein from the *Escherichia coli,* for example.

When grafting the tag sequence on the amino acid residue of the protein, the tagged protein may be prepared by coupling a tag sequence prepared by polypeptide solid-phase synthesis or the like to the purified protein, for example. The protein and the tag sequence can be coupled between the amino group and/or the imino group of the protein and the terminal carboxyl group of the tag sequence and/or between the carboxyl group of the protein and the amino group and/or the imino group of the tag sequence by treating a mixture of the protein and the tag sequence using a carbodiimide reagent such as N-ethyl-N'-(dimethylaminopropyl)carbodiimide (EDC), for example.

### 1.2. Support

The support on which the tagged protein is immobilized has a functional group that is chemically bound to the tagged protein. The functional group is at least one functional group selected from a carboxyl group, an epoxy group, and a tosyl group. Since the tag sequence includes a basic amino acid, the functional group included in the support efficiently reacts with the amino group and/or the imino group included in the basic amino acid in the tag sequence so that the protein is chemically bound to the support via the tag sequence. As a result, the tagged protein can be efficiently immobilized on the support. This is considered to contribute to an increase in the amount of protein immobilized.

The tag sequence includes a sequence that includes three consecutive basic amino acid molecules. Since a basic amino acid has an amino group and/or an imino group, the tag sequence has a high density of the amino group and/or the imino group included in the basic amino acids so that the amino group and/or the imino group has high reactivity (described later). Therefore, since the amino group and/or the imino group included in the basic amino acid of the tag sequence can preferentially react with the functional group of the support as compared with amino groups and the like included in the site of the tagged protein other than the tag sequence, the effects on the properties of the protein can be reduced. This is considered to contribute to maintaining the properties of the immobilized protein. A tagged protein and a protein-immobilized support in which the tagged protein is chemically bound to the support with high efficiency can be obtained by the above-described method.

The functional group included in the support may be chemically introduced by copolymerization, graft polymerization, coupling, a plasma process, or the like when preparing the support. Alternatively, the functional group may be physically introduced by mixing, coating, or the like. It is preferable to chemically introduce the functional group from the viewpoint of functional group introduction efficiency.

### 1.3. Immobilization of tagged protein on support

The tagged protein is chemically bound to the support by reacting the functional group of the support with the amino group and/or the imino group included in the tagged protein.

When the support has a carboxyl group, the carboxyl group and the amino group and/or the imino group included in the tagged protein may be bound by amine coupling using a carbodiimide reagent (e.g., EDC), for example.

In the method of producing the protein-immobilized support according to this embodiment, when using a tagged protein having a tag sequence including a sequence that includes three or more consecutive basic amino acids, the coupling efficiency with the functional group of the support can be increased to a large extent so that the amount of protein immobilized on the support can be significantly increased. As a result, a protein (e.g., Protein G or Protein A) that has been difficult to immobilize on a support in a sufficient amount can be efficiently immobilized on the support.

In the method of producing the protein-immobilized support according to this embodiment, the coupling efficiency with the functional group of the support can be further increased by utilizing a sequence that includes three or more consecutive amino acids that are lysine, arginine, or histidine as the tag sequence. Specifically, when identical amino acids are consecutively present in the shape of a cluster, the reactivity of the amino acid residues increases. The reasons for this phenomenon are considered to be as follows. Specifically, when using a tag sequence that includes three or more consecutive amino acids that are lysine, arginine, or histidine, the density of the amino groups or the imino groups involved in the reaction locally increases so that the interaction with the functional group of the support increases and the amino acids of the tag sequence have a conformation appropriate for the reaction.

In the method of producing the protein-immobilized support according to this embodiment, when the support has at least one functional group selected from a carboxyl group, an epoxy group, and a tosyl group (particularly an epoxy group and/or a tosyl group), the amount of protein immobilized on the support can be increased. In this case, the tagged protein and the support can be bound by dispersing the tagged protein and the support in an appropriate solvent, and mixing the dispersion for a given period of time.

The material for the support is not particularly limited. For example, an organic material, an inorganic material, glass, a metal, or a composite of these materials may be used.

The support may be in the shape of particles, a film, a slide, a disk, a plate, fibers, or a tube, for example.

When the support is in the shape of particles, the support is preferably magnetic particles. In this case, the support may include a magnetic material inside of or on the surface of the particles. When the support is magnetic particles, it is preferable that the magnetic material be contained only inside the particles (i.e., not exposed on the surface). If the support is magnetic particles, the particles can be subjected to solid-liquid separation using a magnetic effect.

The internal structure of the magnetic particles may be homogeneous or heterogeneous. The magnetic particles are preferably heterogeneous particles containing superparamagnetic fine particles that exhibit low residual magnetization. It is preferable that the magnetic particles include an organic substance since precipitation of the magnetic particles in water can be delayed due to a decrease in specific gravity so that the magnetic particles can be easily dispersed in water.

Examples of the magnetic particles having a heterogeneous internal structure include (I) particles in which magnetic fine particles are dispersed in a continuous phase of a non-magnetic organic substance such as an organic polymer, (II) particles including a core formed of a secondary aggregate of magnetic fine particles and a shell formed of a non-magnetic organic substance such as an organic polymer, (III) particles including nuclear particles formed of a non-magnetic material such as an organic polymer, a secondary aggregate layer (magnetic material layer) formed of superparamagnetic fine particles provided on the surface of the nuclear particles, and an organic polymer layer that forms the outer layer of the magnetic material layer, and the like. Among these, it is preferable to use the particles (III) including the organic polymer layer as the outer layer of the nuclear particles including the secondary aggregate layer formed of superparamagnetic fine particles (the nuclear particles including the secondary aggregate layer formed of superparamagnetic fine particles are hereinafter referred to as "mother particles"). The organic polymer layer may include two or more polymer layers.

The organic polymer that is used for the above-mentioned particles and forms the outermost surface of the particles preferably has at least one functional group selected from a carboxyl group, an epoxy group, and a tosyl group excluding the core of the core-shell type particles. The interface between the nuclear particles and the outer layer (magnetic material layer) and the interface between the magnetic material layer and the outer layer (organic polymer layer) may be in a state in which the components of these layers are mixed.

The particles (I) are preferably produced by the method disclosed in JP-A-9-208788, for example. The particles (III) are preferably produced by the method disclosed in JP-A-2004-205481, for example.

As the magnetic material, a ferrite (e.g., ferric oxide (Fe₃O₄) and gamma-iron oxide (gamma-Fe₂O₃)), a metal (e.g., iron, manganese, cobalt, and chromium), an alloy of such a metal, or the like may be used. A support that is substantially superparamagnetic can be obtained by utilizing a magnetic material having an average particle diameter of 30 nm or less.

The content of the magnetic material is preferably 10 wt% or more, and more preferably 20 to 80 wt%, based on the weight of the entire particle. If the content of the magnetic material is 10 wt% or less based on the weight of the entire particle, separation may take time since an excellent magnetic separation capability may not be obtained. If the content of the magnetic material is 80 wt% or more based on the weight of the entire particle, the amount of magnetic material exposed on the surface of the particle may increase.

When the support is in the shape of particles, the diameter of the particles (support particles) is not particularly limited, but is normally 10 nm to 10 mm. The particle diameter is determined by a laser diffraction-scattering method. The shape of the particles need not be spherical, but may be an irregular shape (e.g., needle shape).

The protein-immobilized support according to this embodiment may be a support in which Protein A or Protein G (i.e., a protein having affinity for an antibody molecule) is immobilized on a support such as a sepharose gel, for example. Since the amount of Protein A or Protein G immobilized on the support can be increased as compared with a related-art support, the antibody purification capability can be increased.

In the method of producing the protein-immobilized support according to this embodiment, the protein immobilized on the support may be an antibody, fine particles formed of a polymer latex or the like may be used as the support, and a support obtained by binding the antibody to the fine particles may be used as an immunodiagnosis support for a sandwich ELISA method or the like. In this case, the amount of antibody immobilized on the support can be increased. Therefore, since the measurement limit concentration of the analysis target antigen can be increased on the low concentration side and the high concentration side, a diagnostic reagent that has a wide dynamic range for the concentration of the measurement target and enables a quick test can be obtained.

When immobilizing various proteins or antibodies on a chip to produce a protein chip or an antibody chip, a chip in which a variation in immobilization amount between the proteins is small and which enables high-accuracy evaluation can be obtained.

### 2. Examples

Examples according to the invention are described below. Note that the invention is not limited to the following examples.

### 2.1. Synthesis Example 1 (synthesis of support (magnetic particles) having carboxyl group)

2 parts by mass of a 75% di(3,5,5-trimethylhexanoyl) peroxide solution ("Peroyl 355-75(S)" manufactured by NOF Corporation) and 20 parts by mass of a 1% sodium dodecyl sulfate aqueous solution were mixed, and finely emulsified using an ultrasonic disperser. The emulsion was added to a reactor containing 13 parts by mass of polystyrene particles having a particle diameter of 0.77 micrometers and 41 parts by mass of water. The mixture was then stirred at 25°C for 12 hours. 96 parts by mass of styrene and 4 parts by mass of divinylbenzene were emulsified in another vessel using 400 parts by mass of a 0.1% sodium dodecyl sulfate aqueous solution. The resulting emulsion was added to the reactor. After stirring the mixture at 40°C for two hours, the mixture was heated to 75°C and polymerized for eight hours. After cooling the resulting product to room temperature, the particles were separated by centrifugation, washed with water, dried, and then ground to obtain core particles. The number average particle diameter of the core particles was 1.5 micrometers.

Acetone was added to an oily magnetic fluid ("EXP series" manufactured by Ferrotec Corporation) to precipitate particles. The particles were then dried to obtain ferrite-based magnetic fine particles (average primary particle diameter: 0.01 micrometers) having a hydrophobized surface.

15 g of the core particles and 15 g of the magnetic fine particles were thoroughly mixed using a mixer. The mixture was processed using a hybridization system ("NHS-0" manufactured by Nara Machinery Co., Ltd.) at a peripheral blade (stirring blade) speed of 100 m/sec (16,200 rpm) for five minutes to obtain mother particles (number average particle diameter: 2.0 micrometers) having a magnetic material layer formed of the magnetic fine particles on the surface.

A 1-liter separable flask was charged with 375 g of an aqueous solution containing 0.25 wt% of sodium dodecylbenzenesulfonate and 0.25 wt% of a nonionic emulsifying agent ("Emulgen 150" manufactured by Kao Corporation) (hereinafter referred to as "dispersant aqueous solution"), followed by the addition of 15 g of the mother particles having the magnetic material layer. The mother particles were then dispersed using a homogenizer, and heated to 60°C. A pre-emulsion prepared by dispersing 27 g of methyl methacrylate, 3 g of trimethylolpropane trimethacrylate (hereinafter referred to as "TMP"), and 0.6 g of di(3,5,5-trimethylhexanoyl) peroxide ("Peroyl 355" manufactured by NOF Corporation) in 150 g of the dispersant aqueous solution was added dropwise to the separable flask, of which the temperature was controlled at 60°C, over one and a half hours.

After the addition, the mixture was stirred at 60°C for one hour. A pre-emulsion prepared by dispersing 13.5 g of cyclohexyl methacrylate, 1.5 g of methacrylic acid, and 0.3g of di(3,5,5-trimethylhexanoyl) peroxide ("Peroyl 355" manufactured by NOF Corporation) in 75 g of the dispersant aqueous solution was then added dropwise to the separable flask, of which the temperature was controlled at 60°C, over one and a half hours. After heating the mixture to 75°C, the mixture was polymerized for two hours to complete the reaction.

The particles in the separable flask were magnetically separated, and repeatedly washed with distilled water. Magnetic particles having a carboxyl group (hereinafter referred to as "particles A") were thus obtained.

### 2.2. Synthesis Example 2 (synthesis of support (magnetic particles) having epoxy group)

Magnetic particles having an epoxy group (hereinafter referred to as "particles B") were obtained in the same manner as in Synthesis Example 1, except for using 13.5 g of GMA and 1.5 g of TMP instead of 13.5 g of cyclohexyl methacrylate and 1.5 g of methacrylic acid, respectively.

### 2.3. Synthesis Example 3 (synthesis of support (magnetic particles) having tosyl group)

A 1-liter separable flask was charged with 5 g of the particles B obtained by freeze-drying. After the addition of 60 ml of 1 mol/l sulfuric acid, the mixture was stirred at 60°C for six hours. The particles in the separable flask were magnetically separated, and repeatedly washed with distilled water.

Magnetic particles having a 2,3-dihydroxypropyl group were thus obtained. 1.0 g of dry particles obtained by freeze-drying the particles were dispersed in 8 ml of pyridine. After the addition of 0.2 g of p-tosyl chloride, the mixture was stirred at room temperature for two hours. After completion of the reaction, the particles were magnetically separated, washed four times with acetone, and washed four times with distilled water to obtain magnetic particles in which the 2,3-dihydroxypropyl group was tosylated (hereinafter referred to as "particles C"). The number average particle diameter of the magnetic particles (particles C) was 2.9 micrometers.

### 2.4. Example 1

The amount of protein immobilized on the particles A having a carboxyl group was evaluated using the following four types of proteins. Specifically, (i) glutathione-S-transferase having a 6-histidine-tag containing six consecutive histidine molecules at the N-terminal ("His6-GST" manufactured by Upstate Biotechnology, catalog No. 12-350, molecular weight: 27 kDa), (ii) Akt1 having a 6-histidine-tag at the N-terminal ("His6-Akt1" manufactured by Upstate Biotechnology, catalog No. 14-279, molecular weight: 59 kDa), (iii) ubiquitin having a 6-histidine-tag at the N-terminal ("His6-Ubiquitin" manufactured by AFFINITI Research Products Ltd., catalog No. UW8610, molecular weight: 9.4 kDa), and (iv) ubiquitin having a 10-histidine-tag containing ten consecutive histidine molecules at the N-terminal ("His10-Ubiquitin" manufactured by R&D Systems, Inc., catalog No. 701-UB, molecular weight: 10 kDa) were used.

The solvent of each protein was replaced by a sodium phosphate buffer solution (10 mM, pH: 7.0) by ultrafiltration, and the concentration was adjusted to 1.0 mg/ml based on the absorbance. The amount of each protein immobilized on the particles A was evaluated as follows.

Specifically, 1 mg of the particles A was removed from the particle A liquid dispersion using a test tube. After separating the particles using a test tube magnetic stand, the supernatant liquid was removed. The particles were then dispersed in 100 microliters of a 0.1M MES buffer solution (pH: 5.0). After the addition of 5 microliters of an EDC MES solution (concentration: 10 mg/ml), the mixture was reacted at 25°C for 30 minutes. After separating the particles using the magnetic stand, the solvent was removed. 100 microliters of the MES buffer solution was then added to the particles. After the addition of 20 microliters of a protein solution, the mixture was reacted at 25°C for three hours with shaking. The particles were then washed four times with 0.5 ml of a washing liquid (0.05% Tween20 surfactant-containing PBS buffer) to obtain protein-immobilized magnetic particles.

The amount of protein immobilized on the magnetic particles was measured by BCA assay (protein determination method). The concentration was calculated based on the measurement target protein solution as a standard substance. The results are shown in Table 1.

### 2.5. Comparative Example 1

As a comparative example of Example 1, the amount of protein immobilized on the particles A was measured in the same manner as in Example 1 using the following four types of proteins. Specifically, (i) glutathione-S-transferase ("GST" manufactured by Sigma, product No. G5663, molecular weight: 26 kDa), (ii) Akt1 having a GST-tag ("GST-Akt1" manufactured by Abnova Corporation, catalog No. H00000207-P01, molecular weight: 79 kDa), (iii) ubiquitin ("Ubiquitin" manufactured by Novus Biologicals, Inc., catalog No. NB800-PC40, molecular weight: 8.5 kDa), and (iv) ubiquitin having a GST-tag at the N-terminal ("GST-Ubiquitin" manufactured by Novus Biologicals, Inc., catalog No. NB800-PC42, molecular weight: 35 kDa) were used.

The solvent of each protein was replaced by a sodium phosphate buffer solution (10 mM, pH: 7.0) by ultrafiltration, and the concentration was adjusted to 1.0 mg/ml based on the absorbance. The results are shown in Table 1.

### 2.6. Example 2

The amount of protein immobilized on the particles B having an epoxy group was evaluated using the four types of proteins used in Example 1. Specifically, 1 mg of the particles B was removed from the particle B liquid dispersion using a test tube. After separating the particles using a test tube magnetic stand, the supernatant liquid was removed. The particles were then dispersed in a 0.1M boric acid buffer solution (pH: 9.5). After the addition of 20 microliters of a protein solution, the mixture was reacted at 37°C for 24 hours with shaking. The particles were then washed four times with 0.5 ml of a washing liquid (0.05% Tween20 surfactant-containing PBS buffer) to obtain protein-immobilized magnetic particles. The amount of protein immobilized on the magnetic particles was measured in the same manner as in Example 1. The results are shown in Table 2.

### 2.7. Comparative Example 2

As a comparative example of Example 2, the amount of protein immobilized on the particles B having an epoxy group was evaluated in the same manner as in Example 2 using the four types of proteins used in Comparative Example 1. The results are shown in Table 2.

### 2.8. Example 3

Each protein was immobilized on the particles and the amount of protein immobilized on the particles was measured in the same manner as in Example 2, except for using the particles C having a tosyl group instead of the particles B. The results are shown in Table 3.

### 2.9. Comparative Example 3

As a comparative example of Example 3, each protein was immobilized on the particles and the amount of protein immobilized on the particles was measured in the same manner as in Comparative Example 2, except for using the particles C having a tosyl group instead of the particles B. The results are shown in Table 3.

**TABLE 1**

| Amount of protein immobilized per mg of particles A having a carboxyl group | | | |
|---|---|---|---|
| Example 1 | | Comparative Example 1 | |
| His6-GST | 18 micrograms | GST | 10 micrograms |
| His6-Akt1 | 14 micrograms | GST-Akt1 | 9 micrograms |
| His6-Ubiquitin | 14 micrograms | Ubiquitin | 9 micrograms |
| His10-Ubiquitin | 16 micrograms | GST-Ubiquitin | 11 micrograms |

**TABLE 2**

| Amount of protein immobilized per mg of particles B having an epoxy group | | | |
|---|---|---|---|
| Example 2 | | Comparative Example 2 | |
| His6-GST | 4 micrograms | GST | 2 micrograms |
| His6-Akt1 | 3 micrograms | GST-Akt1 | 1 micrograms |
| His6-Ubiquitin | 4 micrograms | Ubiquitin | 2 micrograms |
| His10-Ubiquitin | 5 micrograms | GST-Ubiquitin | 3 micrograms |

**TABLE 3**

| Amount of protein immobilized per mg of particles C having a tosyl group | | | |
|---|---|---|---|
| Example 3 | | Comparative Example 3 | |
| His6-GST | 8 micrograms | GST | 5 micrograms |
| His6-Akt1 | 6 micrograms | GST-Akt1 | 3 micrograms |
| His6-Ubiquitin | 5 micrograms | Ubiquitin | 3 micrograms |
| His10-Ubiquitin | 6 micrograms | GST-Ubiquitin | 3 micrograms |

### 2.10. Example 4

In this example, the effect of the tag sequence on the amount of protein immobilized on the support was examined using Protein G. The molecule described in Bengt Guss et al. (1986), EMBO Journal. 5 (7), pp. 1567 to 1575 was modified and used as Protein G

Specifically, a 6-lysine tag containing six consecutive lysine molecules (hereinafter referred to as "Lys6") was fused to the N-terminal of a sequence (amino acid number: 190 to 384) of Protein G described in the above document to prepare a lysine-tag-fused protein. A gene encoding the sequence (amino acid number: 190 to 384) was obtained according to the method described in the above document. A DNA in which a DNA sequence encoding the N-terminal-side six amino acids is bound to a DNA molecule encoding six lysine molecules was chemically synthesized. The DNA and a DNA encoding C-terminal-side twelve amino acids were used as PCR primers and amplified by PCR to prepare a gene encoding Protein G to which the DNA encoding the 6-lysine-tag was fused. The gene was introduced into an expression vector and expressed by *Escherichia coli,* followed by separation and purification to prepare a tagged protein (Lys6-Protein G) having the target tag sequence (lysine-tag).

The Lys6-Protein G was immobilized on the particles A having a carboxyl group, and the amount of protein immobilized on the particles A was measured in the same manner as in Example 1. The results are shown in Table 4.

The amount of immunoglobulin G (IgG) immobilized on the Lys6-Protein G-immobilized particles was also measured. Specifically, 1 mg of the Lys6-Protein G-immobilized particles was removed from the liquid dispersion of the Lys6-Protein G-immobilized particles using a test tube. After separating the particles using a test tube magnetic stand, the supernatant liquid was removed. The particles were then dispersed in 50 microliters of a 0.1M citric acid-phosphoric acid buffer solution (pH: 5.0). After the addition of 10 microliters of a 0.1M citric acid-phosphoric acid buffer solution (1 mg/ml, pH: 5.0) of human IgG ("I4506" manufactured by Sigma), the mixture was reacted at 25°C for one hour with shaking. After washing unreacted human IgG several times with a 0.1M citric acid-phosphoric acid buffer solution (pH: 5.0), the human IgG captured by the particles was eluted using 50 microliters of a 0.1M citric acid buffer solution (pH: 2.0). The amount of IgG recovered was calculated based on the absorbance. Table 4 shows the amount of IgG bound per mg of the particles.

### 2.11. Comparative Example 4

A Protein G molecule corresponding to the sequence (amino acid number: 190 to 384) of Protein G described in the document referred to in Example 4 was prepared. A gene encoding the sequence (amino acid number: 190 to 384) of Protein G was obtained according to the method described in the above document. The gene was introduced into an expression vector, and expressed by *Escherichia coli,* followed by separation and purification to prepare the target Protein G

The Protein G was immobilized on the particles A having a carboxyl group and the amount of protein immobilized on the particles A was measured in the same manner as in Example 1. The results are shown in Table 4.

The amount of human IgG bound was also measured in the same manner as in Example 4. The results are shown in Table 4.

**TABLE 4**

| Amount of Protein G immobilized and amount of human IgG bonded per mg of particles | | | |
|---|---|---|---|
| Example 4 | | Comparative Example 4 | |
| Amount of Lys6-Protein G immobilized (micrograms/mg-bead) | Amount of IgG bound (micrograms/mg-bead) | Amount of Protein G immobilized (micrograms/mg-bead) | Amount of IgG bound (micrograms/mg-bead) |
| 7.0 | 16 | 1.5 | 2.8 |

As shown in Tables 1 to 4, when preparing a tagged protein using a tag sequence including a sequence containing three or more consecutive basic amino acids, and immobilizing the tagged protein on the support, the amount of protein immobilized on the support could be increased.

Table 1 shows the amount of protein immobilized per mg of the particles A having a carboxyl group. As shown in Table 1, the amount of protein immobilized on the support (particles having a carboxyl group) was larger for the protein having the tag sequence (His-tag) as compared with the protein that did not have the tag sequence (His-tag) irrespective of the type of protein.

Table 2 shows the amount of protein immobilized per mg of the particles B having an epoxy group. As shown in Table 2, the amount of protein immobilized on the support was larger for the protein having the tag sequence (His-tag) as compared with the protein that did not have the tag sequence (His-tag) irrespective of the type of protein.

Table 3 shows the amount of protein immobilized per mg of the particles C having a tosyl group. As shown in Table 3, the amount of protein immobilized on the support (particles having a tosyl group) was larger for the protein having the tag sequence (His-tag) as compared with the protein that did not have the tag sequence (His-tag) irrespective of the type of protein.

Table 4 shows the amounts of Protein G and Lys6-Protein G immobilized per mg of the particles having a carboxyl group and the amount of human IgG bonded per mg of the particles having a carboxyl group. As shown in Table 4, the amount of Protein G immobilized on the support (particles having a carboxyl group) was increased when using the tagged protein obtained by fusing the Lys6-tag to Protein G Since the amount of IgG bound to the particles on which the tagged protein (Lys6-Protein G) was immobilized was larger than that of the particles on which the protein (Protein G) that did not have the Lys6-tag was immobilized, it was confirmed that the performance of the protein-immobilized support could be improved by utilizing the tagged protein (Lys6-Protein G) obtained by fusing the tag sequence (Lys6-tag) to the protein (Protein G).

As shown in Table 4, it was confirmed that the amount of protein immobilized on the support was increased when using Protein G so that the performance of the protein-immobilized support indicated by the amount of IgG bonded could be improved.

## Claims

1. A method of producing a protein-immobilized support comprising immobilizing a protein having a tag sequence on a support, the tag sequence including a sequence that includes three or more consecutive basic amino acids that are lysine, arginine, or histidine,
wherein the support has at least one functional group selected from a carboxyl group, an epoxy group, and a tosyl group; and
wherein the amino group(s) and/or the imino group(s) included in the basic amino acids of the tag sequence react with the functional group of the support.

2. The method of producing a protein-immobilized support according to claim 1, wherein the support has at least one functional group selected from an epoxy group and/or a tosyl group.

3. The method of producing a protein-immobilized support according to claim 1 or 2, wherein the tag sequence is a histidine-tag.

4. The method of producing a protein-immobilized support according to any one of claims 1 to 3, wherein the support is magnetic particles.

5. The method of producing a protein-immobilized support according to any one of claims 1 to 4, wherein the tag sequence entirely includes basic amino acids.

6. A protein-immobilized support obtained by the method of producing a protein-immobilized support according to any one of claims 1 to 5.

## Patentansprüche

1. Verfahren zur Herstellung eines Protein-immobilisierten Trägers, das Immobilisieren eines Proteins mit einer Tag-Sequenz auf einem Träger umfasst, wobei die Tag-Sequenz eine Sequenz beinhaltet, die drei oder mehr konsekutive basische Aminosäuren beinhaltet, die Lysin, Arginin oder Histidin sind,
wobei der Träger zumindest eine funktionelle Gruppe aufweist, die aus einer Carboxylgruppe, einer Epoxidgruppe und einer Tosylgruppe ausgewählt ist; und
wobei die Aminogruppe(n) und/oder die Iminogruppe(n), die in den basischen Aminosäuren der Tag-Sequenz beinhaltet sind, mit der funktionellen Gruppe des Trägers reagiert/reagieren.

2. Verfahren zur Herstellung eines Protein-immobilisierten Trägers nach Anspruch 1, wobei der Träger zumindest eine funktionelle Gruppe aufweist, die aus einer Epoxidgruppe und/oder einer Tosylgruppe ausgewählt ist.

3. Verfahren zur Herstellung eines Protein-immobilisierten Trägers nach Anspruch 1 oder 2, wobei die Tag-Sequenz ein Histidin-Tag ist.

4. Verfahren zur Herstellung eines Protein-immobilisierten Trägers nach einem der Ansprüche 1 bis 3, wobei der Träger magnetische Teilchen ist.

5. Verfahren zur Herstellung eines Protein-immobilisierten Trägers nach einem der Ansprüche 1 bis 4, wobei die Tag-Sequenz lediglich basische Aminosäuren beinhaltet.

6. Protein-immobilisierter Träger, der durch das Verfahren zur Herstellung eines Protein-immobilisierten Trägers nach einem der Ansprüche 1 bis 5 erhalten ist.

## Revendications

1. Procédé de production d'un support portant une protéine immobilisée sur sa surface comprenant l'immobilisation d'une protéine pourvue d'une séquence d'étiquette sur un support, la séquence d'étiquette contenant une séquence qui comprend trois acides aminés basiques consécutifs ou plus qui sont de type lysine, arginine, ou histidine,
dans lequel le support comporte au moins un groupe fonctionnel choisi parmi un groupe carboxyle, un groupe époxy, et un groupe tosyle ; et
dans lequel le ou les groupes amino et/ou le ou les groupes imino contenus dans les acides aminés basiques de la séquence d'étiquette réagissent avec le groupe fonctionnel du support.

2. Procédé de production d'un support portant une protéine immobilisée sur sa surface selon la revendication 1, dans lequel le support comporte au moins un groupe fonctionnel choisi parmi un groupe époxy et/ou un groupe tosyle.

3. Procédé de production d'un support portant une protéine immobilisée sur sa surface selon la revendication 1 ou 2, dans lequel la séquence d'étiquette est une étiquette histidine.

4. Procédé de production d'un support portant une protéine immobilisée sur sa surface selon l'une quelconque des revendications 1 à 3, dans lequel le support est constitué de particules magnétiques.

5. Procédé de production d'un support portant une protéine immobilisée sur sa surface selon l'une quelconque des revendications 1 à 4, dans lequel la séquence d'étiquette est entièrement constituée d'acides aminés basiques.

6. Support portant une protéine immobilisée sur sa surface, obtenu par le procédé de production d'un support portant une protéine immobilisée sur sa surface selon l'une quelconque des revendications 1 à 5.
